# EUROPEAN PATENT SPECIFICATION

(11) **EP 1 755 561 B1**
(45) Date of publication and mention of the grant of the patent: **23.11.2016**
(21) Application number: 05746586.6
(22) Date of filing: 01.06.2005
(51) Int. Cl.: A61K 9/20, A61K 9/22, A61K 9/28, A61K 31/192, A61K 31/495, A61K 31/55, A61K 45/06

(54) **MULTI-LAYER TABLET COMPRISING NON-STEROIDAL ANTI-INFLAMMATORY DRUGS DECONGESTANTS AND NON-SEDATING ANTIHISTAMINES**
MEHRSCHICHTIGE TABLETTE MIT NICHTSTEROIDALEN ANTIRHEUMATIKA, ABSCHWELLENDEN MITTELN UND NICHT-SEDIERENDEN ANTIHISTAMINIKA
COMPRIME MULTICOUCHE COMPRENANT DES MEDICAMENTS ANTI-INFLAMMATOIRES NON STEROIDIENS, DES DECONGESTIONNANTS ET DES ANTIHISTAMINIQUES NON SEDATIFS

(30) Priority: 02.06.2004 US 859256
(43) Date of publication of application: 28.02.2007
(73) Proprietor: Wyeth LLC, New York, NY 10017-5755 (US)
(72) Inventor: BERLIN, Roger, Mendham, NJ 07945 (US); RAMSEY, Peter, Midlothian, VA 23114 (US); USAYAPANT, Arunya, Ivanhoe, IL 60060 (US); FUBARA, Josephine, Richmond, VA 23233 (US); POXON, Scott, Mechanicsville, VA 23116 (US); BUBNIS, William, Mechanicsville, VA 23116 (US); GIAMALVA, David, Glen Allen, VA 23060 (US); STRODE, John, Mechanicsville, VA 23118 (US); BACHERT, John, Richmond, VA 23235 (US); WILLIAMS, Michael, Midlothian, VA 23113 (US)
(74) Representative: Pfizer
(86) International application number: PCT/US2005/019200
(87) International publication number: WO 2005/120465

(56) References cited:
- EP-A- 0 348 683
- WO-A1-95/07103
- WO-A1-03/089007
- CA-A1- 2 084 028
- US-A- 5 462 747
- US-B1- 6 210 710

## Description

### CROSS REFERENCE TO RELATED APPLICATIONS

This application claims priority from U.S. Application 10/859,256, filed June 2, 2004 entitled "COMPOSITIONS OF NON-STEROIDAL ANTI-INFLAMMATORY DRUGS, DECONGESTANTS AND ANTIHISTAMINES".

### FIELD OF THE INVENTION

The present invention relates to improved dosage forms of pharmaceuticals for treating rhinitis associated with allergies and colds.

### BACKGROUND OF THE INVENTION

### DECONGESTANTS AND ANTIHISTAMINES

Rhinitis refers to an inflammatory disorder of the nasal passages. The symptoms of rhinitis typically consist of sneezing, rhinorrhea, nasal congestion, and increased nasal secretions. Untreated rhinitis may lead to other disorders including infection of the sinuses, ears and lower respiratory tract.

Two types of oral medication are commonly used to treat rhinitis: decongestants and antihistamines. Decongestants and antihistamines differ in mechanism of action, therapeutic effects, and side effects. It is common practice to combine the use of decongestants and antihistamines to bring about more complete symptom relief of rhinitis than is possible with either entity alone.

Decongestants commonly used to treat rhinitis include the sympatomimetic agents pseudoephedrine and phenylephrine. These agents act to constrict vessels in the nasal mucus membranes and thereby decrease tissue swelling and nasal congestion. Decongestants are found to be better than antihistamines for restoring the patency of congested nasal airways. Nasal decongestants are stimulatory. Decongestants, however may produce nervousness, restlessness and insomnia, especially if taken at night.

Histamine is a mediator released from cells which line the walls of the nasal mucous membranes (mast cells). When released, histamine is known to bind to local receptors and thereby cause sneezing, nasal itching, swelling of the nasal membranes, and increased nasal secretions. Antihistamines relieve these effects, albeit by a different mechanism than decongestants. Antihistamines block the binding of histamine to histamine receptors in the nasal membranes. Side effects of antihistamines frequently include impairment of mental acuity and sedation.

Combinations of decongestants and antihistamines employ two mechanistic approaches, and have been shown to offer more complete relief of rhinitis symptoms than therapy with either component alone. Currently, many cold and allergy relief products contain both. Incorporation of decongestant and sedating antihistamine into a single dosage unit balances stimulation and sedation of the components. However, some individuals vary in their sensitivity to either the decongestant or the antihistamine. Consequently, some individuals experience an irritability and/or sedation with these combinations.

Examples of commercial formulations containing decongestant and sedating antihistamine include:
1. CHLOR-TRIMETON™ 4 hour Allergy/Decongestant which contains 4 mg of chlorpheniramine (sedating antihistamine) and 60 mg pseudoephedrine sulfate (stimulating decongestant), and which is recommended to be taken every 4 to 6 hours (1/2 this dosage for children 6 to under 12);
2. CHLOR-TRIMETON™ 12 hour Allergy/Decongestant which contains 8 mg of chlorpheniramine (sedating antihistamine) and 120 mg pseudoephedrine sulfate (stimulating decongestant), and which is recommended to be taken every 12 hours (adults and children over 12 years of age only);
3. BROMFED^{™} Tablets which contains 4 mg of brompheniramine (sedating antihistamine) and 60 mg pseudoephedrine sulfate (stimulating decongestant), and which is recommended to be taken every 4 to 6 hours (1/2 this dosage for children 6 to under 12);
4. BROMFED^{™} Capsules which contains 12 mg of brompheniramine (sedating antihistamine) and 120 mg pseudoephedrine sulfate (stimulating decongestant), and which is recommended to be taken every 12 hours (adults and children over 12 years of age only);
5. BENADRYL^{™} Allergy Decongestant Tablets which contains 25 mg of diphenhydramine hydrochloride (sedating antihistamine) and 60 mg pseudoephedrine sulfate (stimulating decongestant), and which is recommended to be taken by adults and children over 12 years of age every 4 to 6 hours, not to exceed 4 tablets in 24 hours; and
6. TAVIST-D™ Tablets which contains 1.34 mg clemastine fumarate (sedating antihistamine) and 75 mg phenylpropanolamine hydrochloride (stimulating decongestant), and which is recommended to be taken every 12 hours (adults and children over 12 years of age only).

Formulations have been commercialized that incorporate both a decongestant and a non-sedating antihistamine into a single dosage unit. While such formulations offer the advantage in being non-sedating, their efficacy does not approach that offered by sedating antihistamines, especially for rhinitis due to colds.

### NON-STEROIDAL ANTI-INFLAMMATORY DRUGS

Non-steroidal anti-inflammatory drugs (NSAIDS) are ideally suited for use in cold formulations for their analgesic, anti-inflammatory, and antipyretic activity and low incidence of side effects. Exemplary cold formulations containing non-steroidal anti-inflammatory agents include Advil Cold and Sinus^{™}, Motrin Cold and Flu^{™}, Motrin 1B Sinus^{™} and Dristan Sinus^{™}, each containing 200mg ibuprofen and 30 mg pseudoephedrine.

U.S. Patent 5,025,019 teaches pharmaceutical compositions and methods of using a composition containing a non-steroidal anti-inflammatory drug in combination with at least one other active component selected from an antihistamine, decongestant, cough suppressant or expectorant.

EP 348683 discloses immediate-release multi-layered tablets comprising ibuprofen, an antihistamine and decongestant.

While these combination products provide effective symptom treatment of rhinitis due to cold and allergy, they do not alleviate the side effects of decongestants and antihistamines in sensitive individuals. Further these combination products do not provide extended non-drowsy relief for treating the range of rhinitis symptoms associated with allergies and /or a cold. Thus, there remains a need in the art for improved compositions and methods for treating symptoms of rhinitis with reduced side effects from the treatment and/or with improved release characteristics of the pharmaceutical actives.

### SUMMARY OF THE INVENTION

The present invention is directed to a pharmaceutical composition which includes an effective amount of each of a non-steroidal anti-inflammatory drug (NSAID), a decongestant, and an antihistamine.

In one embodiment the pharmaceutical composition comprises naproxen, loratadine and pseudoephedrine wherein the naproxen, loratadine and pseudoephedrine are in a bi-layer tablet and wherein the naproxen and loratadine are in a first layer of the bi-layer tablet and pseudoephedrine suitably modified to extend the release profile to substantially match the release profiles of naproxen and loratadine is in a second layer of the bi-layer tablet.

Thus, according to the invention, there is provided a pharmaceutical composition comprising naproxen, loratadine and pseudoephedrine wherein the naproxen, loratadine and pseudoephedrine are in a bi-layer tablet and wherein the naproxen and loratadine are in a first, immediate release layer of the bi-layer tablet and the pseudoephedrine is in a second, extended release layer of the bi-layer tablet.

The multi-layer tablet of the invention may be formed by compression of the immediate release matrix and the extended release matrix, by coating one of the immediate release and extended release matrices on the other of the immediate release and extended release matrices, or by creating a tablet within a tablet wherein in one portion is formed from the extended release matrix and the other portion is formed from the immediate release matrix.

The invention further includes a pharmaceutical composition comprising an active pharmaceutical agent and an amount of magnesium oxide wherein the amount of magnesium oxide is sufficient to modify the release profile of the active pharmaceutical agent and a method for preparing the composition.

### BRIEF DESCRIPTION OF FIGURES

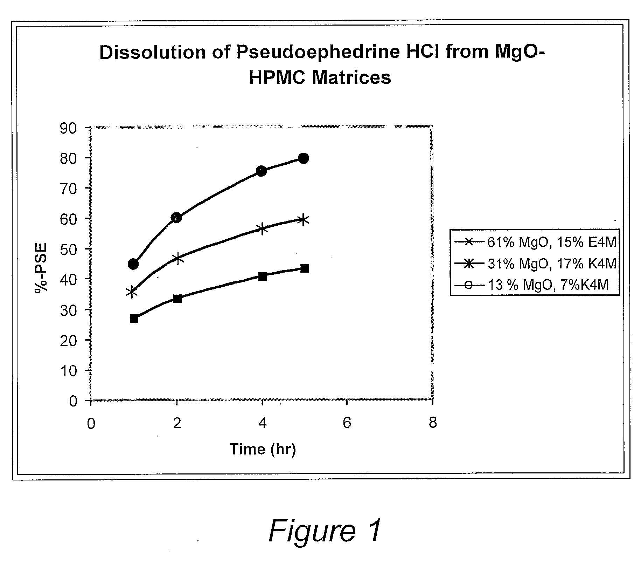

Figure 1 is a graph showing dissolution of pseudoephedrine HCl from MgO-HPMC matrices.

### DETAILED DESCRIPTION OF THE INVENTION

In application US 10/740,386, the inventors disclosed their surprising finding that a pharmaceutical composition comprising a non-steroidal anti-inflammatory agent (NSAID), a decongestant and an antihistamine in which either the antihistamine or decongestant or both were present in an amount less than about 75% of the present approved dosage for the decongestant or the antihistamine and in which the NSAID was present in about 100% of the normal dosage strength provided effective relief of rhinitis symptoms. The compositions of the present invention may use either the dosages discussed and claimed in application US 10/740,386 or conventional dosage amounts.

In the present invention it has been discovered that a combination of a non-steroidal anti-inflammatory agent, a decongestant, and an antihistamine (preferably a non-sedating antihistamine) can be prepared in a multi-layer tablet comprising an immediate release layer and an extended release layer, to provide a composition with an optimized delivery profile and a matched duration of action of the active agents such that their durations of effect are substantially equal. Matched duration of action provides for relief of the range of symptoms for a specified length of time and alleviates the need for taking multiple dosages of various symptom specific drugs on different schedules to maintain relief from the range of rhinitis symptoms. In addition to convenience, this reduces the potential for a patient to take an inappropriate amount or combination of drugs.

"Matched release profile" as used herein means that the duration of action of each of pharmaceutical actives in the pharmaceutical composition as presented to the patient is substantially equal. This may be accomplished by selecting active agents known to have similar durations of action or by modification of one or more of the active agents to alter the duration of action. The modification may mean modification of the pharmaceutical active per se or modification via the use of a matrix (e.g. excipients) used with the pharmaceutical active. Methods of determination of the duration of action or biological profile of a pharmaceutical active are known to those skilled in the art.

The term "extended release matrix" as used herein means a composition comprising at least one pharmaceutical active and excipients wherein either modifications to the pharmaceutical active per se or modifications of the pharmaceutical active as a result of combination with the excipients yields a composition that when administered to a mammal gives a duration of action substantially longer than the unmodified pharmaceutical active.

The term "immediate release matrix" means a composition comprising at least one pharmaceutical active and excipients in which there is no substantial alteration of the release profile of the pharmaceutical active.

In one embodiment the composition of the invention comprises a bi-layer tablet, including an immediate release layer comprising loratadine and naproxen sodium which have comparable biological half-lives which are sufficient to provide a 12-24 hour duration of action without further modification. Not only do naproxen sodium and loratadine have comparable biological half-lives, but also the inventors have surprisingly discovered that the combination of naproxen sodium and loratadine dissolves faster in aqueous media than each separately. In this embodiment, pseudoephedrine HCl, modified to extend the release profile such that the profile is comparable to that of naproxen and loratadine, is used in the extended release layer. The compositions of the invention provide an improved dosage in which extended relief from the range of symptoms manifested by rhinitis is provided in a dosage unit that is preferably taken at 12 or 24 hour intervals.

As used herein, the term "rhinitis" refers to inflammation of the nasal mucous membranes, which could result from a cold, flu, or allergies. Rhinitis may be characterized by one or more cold-like symptoms.

Cold-like symptoms as used herein refers to coryzea, nasal congestion, upper respiratory infections, allergic rhinitis, otitis, sinusitis, and the like. Runny nose and nasal congestion can also be cold symptoms.

The terms "effective amount" or "therapeutically effective amount" of an active agent as provided herein is defined as an amount of the agent at least sufficient to provide the desired therapeutic effect.

The term "normal approved dose" or "approved dose" of an active agent as provided herein is defined as an amount of the active agent that has been approved as safe and effective by the United States Food and Drug Administration for administration in humans in a particular dosage form. An approved dose is thus a dose found in a pharmaceutical product, an amount of active agent per unit dosage form. In the present invention, reference to a ratio of approved doses means doses approved for the same patient population (e.g., adult to adult or pediatric to pediatric), -and approved for the same dosage form (e.g., elixir, tablet, capsule, caplet, controlled release, etc.).

The term "active pharmaceutical agent," "pharmaceutical active," "active agent" and "drug" as used herein should be considered to have the same meaning.

In the practice of the invention, the improved solid dosage forms of the composition of the invention comprises three different drugs from the three different classes of action: non-steroidal anti-inflammatory drugs (NSAIDs), decongestants and antihistamines (preferably non-sedating antihistamines). Selection of a drug from each of these classes is desirable to address the range of symptoms manifested by rhinitis associated with allergies and colds. These symptoms include sneezing, runny nose, itchy watery eyes, itchy nose and throat, sinusitis, nasal congestion, sinus pressure, minor aches, pains and headaches. It is desirable that the drugs selected either be similar in duration of action or that they be modified to have a similar duration of action.

Modifying the duration of action of one pharmaceutical active in the presence of a different pharmaceutical active which is preferentially unmodified can be problematic. The inventors have found that use of a multi-layer tablet facilitates accommodating use of release modifying agents with one or more active agents while minimizing impact of release modifying agents on active agents in other layers of the multi-layer tablet. The use of two or more layers in the tablet also allows for optimization of the matrix for each of the three drug components. Further, in some embodiments separation of active agents into two or more layers may facilitate reduction of undesirable interactions between components. In a preferred embodiment comprising loratadine, naproxen sodium and pseudoephedrine, loratadine and naproxen sodium have a suitable release profile unmodified, while the duration of action of unmodified pseudoephedrine is much shorter than that of loratadine and naproxen sodium. Accordingly, the unmodified loratadine and naproxen sodium may be combined in an immediate release layer while pseudoephedrine may be modified to extend release and included in a second layer of the tablet.

Methods known to those skilled in the art, including either dry or wet granulation methods, may be used to prepare the matrix of each layer of the multi-layer tablet. The thus prepared matrices may be used to form the layers of the multi-layer tablet. The layers may be formed from the matrices using direct compression processes, coating methods, and/or by the so called "tablet with-in a tablet" method. The use of the term "multi-layer tablet" is taken to mean a tablet with two or more layers of distinct compositions. The multi-layer tablet may be formed by any method that yields such a result, including compression using a two or three layer press or through compression using a tablet within a tablet press.

### ANTIHISTAMINES

The term "antihistamine", as used in connection with treating nasal symptoms associated with allergy or cold, generally refers to histamine H₁ receptor antagonists. Numerous chemical substances are known to have histamine H₁ receptor antagonist activity. Many useful compounds can be classified as ethanolamines, ethylenediamines, alkylamines, phenothiazines or piperidines. Representative H₁ receptor antagonists, include, without limitation: astemizole, azatadine, azelastine, acrivastine, brompheniramine, chlorpheniramine, clemastine, cyclizine, carebastine, cyproheptadine, carbinoxamine, descarboethoxyloratadine (also known as SCH-34117), desloratadine doxylamine, dimethindene, ebastine, epinastine, efletirizine, fexofenadine, hydroxyzine, ketotifen, loratadine, levocabastine, mizolastine, mequitazine, mianserin, noberastine, meclizine, norastemizole, picumast, pyrilamine, promethazine, terfenadine, tripelennamine, temelastine, trimeprazine and triprolidine. Other compounds can readily be evaluated to determine activity at H₁ receptors by known methods, including specific blockade of the contractile response to histamine of isolated guinea pig ileum.

Of the foregoing histamine H₁ receptor antagonists, loratadine is specifically exemplified herein. This antihistamine is particularly suitable because of its relatively long biological half-life, allowing it to be effective for 12 hours with a single immediate release dose. Loratadine offers the further advantage that it is non-sedating.

The usual adult dosage of loratadine is 5 mg orally every 12 hours or 10 mg every 24 hours (i.e., daily) with a maximum safe dose of 40 mg per day. The usual pediatric dose is 5 mg per day in children 2-5 years of age and 10 mg per day in older children (8-12 years of age). In accordance with some embodiments of the present invention, the usual adult dose may be reduced to about 2.5 mg to 3.75 mg every 12 hours, or to about 5 mg to 7.5 mg daily, up to a maximum dose of about 30 mg. Similarly, in some embodiments of the invention, the pediatric daily dosage is about 2.5 mg to 3.75 mg daily for children 2-5 years of age and 5 mg to 7.5 mg daily for children 8-12 years of age. In a further embodiment, the invention permits combining a pediatric dosage of loratadine with an adult dosage of the NSAID naproxen.

### DECONGESTANTS

The decongestant for use in the pharmaceutical compositions of the present invention is pseudoephedrine.

The usual adult dose of pseudoephedrine is 60 mg every 4-6 hours, up to a maximum of 240 mg per day. The usual pediatric dose of pseudoephedrine is 15 mg every 6 hours, up to a maximum of 60 mg per day for ages 2-5 and 30 mg every 6 hours, up to a maximum of 120 mg per day for ages 6-12. Thus, in some embodiments of the practice of the present invention, the adult dose can be reduced to 45 or 30 mg every 4-6 hours, with a maximum of 120 to 180 mg per day, and the pediatric dose can be reduced to about 11 or 7.5 mg every 6 hours, up to a maximum of 30-45 mg per day. From the foregoing it is apparent that in some embodiments the invention contemplates administering a double or greater pediatric dose of decongestants with a normal adult dose of an NSAID to an adult.

When modification of the release profile (e.g. duration of action) of the decongestant is desired, formulation approaches known to those skilled in the art may be used to modify the biological pharmacokinetic profile of the decongestant. These approaches may include, for example, the use of HPMC (hydroxypropylmethyl cellulose) to modify release of the active from a compacted tablet. Further, the inventors have surprisingly found that the use of HPMC in combination with magnesium oxide allows significant additional control of the release profile of the active active from a compacted tablet. For instance, magnesium oxide may be used to significantly decrease the release rate of commercially available pseudoephedrine from the extended release matrix prepared with HPMC. This approach may be used to minimize premature release of the active ingredient in an extended release matrix as shown in Figure 1.

The use of magnesium oxide to modify the release of pseudoephedrine especially pseudoephedrine prepared with HPMC is exemplary. The inventions have found magnesium oxide to be generally useful for modulating the release profile for other pharmaceutical actives.

### NSAIDS

The non-steroidal anti-inflammatory drug (NSAID) for use in the pharmaceutical compositions of the present invention is naproxen.

With respect to the dosage amount of the non-steroidal anti-inflammatory drugs in the compositions of the invention, although the specific dose will vary depending upon the age and weight of the patient, the severity of the symptoms, the incidence of side effects and the like, for humans, typical effective analgesic amounts of NSAID per dose is about 125-500 mg naproxen; however, greater or lesser amounts may be employed if desired or necessary.

In one embodiment of this invention, naproxen sodium is the NSAID. Naproxen is useful in part due to its long biological half-life (14±1 hours; Reference: Goodman & Gilman's The Pharmacological Basis of Therapeutics, 9^{th} Edition.). Optimally, naproxen sodium is used in combination with an antihistamine with a similar half-life: loratadine. The usual adult dosage of naproxen sodium is 220 mg orally every 12 hours as needed.

It was discovered in this invention that the combination of naproxen sodium and loratadine exhibits a synergistic effect on rates of *in vitro* dissolution of this combination. The solubility of naproxen is enhanced at a low pH in the presence of loratadine and the solubility of loratadine is enhanced at a high pH in the presence of naproxen. Further, in some instances the rate of dissolving appears to be enhanced for the combination as compared to the rates for naproxen and loratadine individually. This may be useful in that greater solubility is an advantage in immediate-release pharmaceutical preparations since the drug cannot be bio-available until it is dissolved.

The inventors anticipate, without wishing to be held to the theory, that similar synergistic solubility behavior would occur between loratadine and other pharmaceutically active carboxylic acids including, but not limited to, ibuprofen, ketoprofen, and their various salt forms; and between naproxen, ibuprofen, ketoprofen or their salts and amines such as chlorpheniramine, brompheniramine, diphenhydramine.

### ANTI-TUSSIVES

Optionally, anti-tussives act on the brain to suppress the cough reflex. Such cough suppressants are used to relieve dry persistent coughs. The most commonly used drugs are dextromethorphan (an NMDA receptor antagonist), codeine and pholcodine (which are opioids). However, one skilled in the art would understand that there are many other well known and common anti-tussives that may be used. The present invention is optionally directed to the use of anti-tussives.

### PHARMACEUTICAL COMPOSITIONS

Compositions of the invention are formulated in a single solid dosage form, consisting of a multi-layer tablet. For purposes of this specification and the accompanying claims, the term "tablet" refers equally to a tablet, a caplet or any other solid dosage form which is suitable for oral administration. Direct compression, dry granulation or wet granulation methods could be used for preparing the matrix of each layer. These methods are known to those skilled in the art.

In an exemplary embodiment, an immediate release matrix comprising loratadine and naproxen is combined in a bi-layer tablet with an extended release matrix comprising pseudoephedrine. This dosage form and composition is suitable for convenient once or twice a day oral administration as the release of pseudoephedrine is modified to match the duration of action of the immediate release matrix actives loratadine and naproxen. In one embodiment, a bi-layer tablet can be formulated for twice daily oral administration, consisting of an immediate release layer containing 5 mg loratadine and 220 mg naproxen sodium with appropriate excipients, and an extended release layer containing 120 mg pseudoephedrine hydrochloride with appropriate excipients.

In the exemplary embodiment comprising loratadine, naproxen and pseudoephedrine, the extended release matrix comprising pseudoephedrine is prepared by combining pseudoephedrine with suitable extended release polymers such as, but not limited to, hydroxypropyl methylcellulose and polyethylene oxide. It is preferable to use magnesium oxide in addition to the extended release polymer to further modify the release of pseudoephedrine. Other acceptable pharmaceutical excipients include, but are not limited to, fillers (such as lactose, mannitol, microcrystalline cellulose, magnesium oxide, and dibasic calcium phosphate) and glidants and/or lubricants (such as magnesium stearate, glycerol behenate, and silicon dioxide).

In the exemplary embodiment comprising loratadine, naproxen and pseudoephedrine, the immediate release matrix may comprise loratadine and naproxen sodium which are combined with various acceptable pharmaceutical excipients, including suitable fillers (such as dibasic calcium phosphate, lactose, microcrystalline cellulose, starch), suitable binders (such as PVP, HPMC, and HPC), suitable disintegrants (such as crospovidone and croscarmellose sodium), and suitable lubricants and/or glidants (such as glycerol behenate, talc, magnesium stearate, and silicon dioxide).

Binders are agents used to impart cohesive qualities to the powdered material. Binders impart cohesiveness to the tablet formulation which insures the tablet remaining intact after compression, as well as improving the free-flowing qualities by the formulation of granules of desired hardness and size. Suitable binder materials include, but are not limited to, starch (including corn starch and pregelatinzed starch), gelatin, sugars (including sucrose, glucose, dextrose, lactose and sorbitol), polyethylene glycol, waxes, natural and synthetic gums, e.g., acacia, tragacanth, sodium alginate, celluloses, and Veegum, and synthetic polymers such as polymethacrylates and polyvinylpyrrolidone.

Lubricants have a number of functions in tablet manufacture. They prevent adhesion of the tablet material to the surface of the dies and punches, reduce interparticle friction, facilitate the ejection of the tablets from the die cavity and may improve the rate of flow of the tablet granulation. Examples of suitable lubricants include, but are not limited to, magnesium stearate, calcium stearate, stearic acid, glyceryl behenate, talc, sodium lauryl sulfate, sodium stearyl fumarate, polyethylene glycol or mixtures thereof. Generally, the lubricant is present in an amount from about 0.25% to about 5% of the weight of the final composition and more specifically from about 0.5 to about 1.5% of the weight of the final composition.

A disintegrant is a substance, or a mixture of substances, added to a tablet to facilitate its breakup or disintegration after administration. Materials serving as disintegrants have been classified chemically as starches, clay, celluloses, aligns, gums and cross-linked polymers. Examples of suitable disintegrants include, but are not limited to, croscarmellose sodium, sodium starch glycolate, starch, magnesium aluminum silicate, colloidal silicon dioxide, methylcellulose, agar, bentonite, alginic acid, guar gum, citrus pulp, carboxymethyl cellulose, microcrystalline cellulose, or mixtures thereof. Generally, the disintegrant is present in an amount from about 0.5% to about 25% of the weight of the final composition and more specifically from about 1% to about 15% of the weight of the final composition.

Glidants are substances which improve the flow characteristics of a powder mixture. Examples of glidants include, but are not limited to, colloidal silicon dioxide, talc or mixtures thereof. Generally, the glident is present in an amount of from about 0.1 % to about 10% of the weight of the final composition and more specifically from 5 about 0.1% to about 5% of the weight of the final composition.

An adsorbent may be, for example, colloidal silicon dioxide, microcrystalline cellulose, calcium silicate or mixtures thereof. Generally, the adsorbent is present in an amount from about 0.05% to about 42% of the weight of the final composition and more specifically from about 0.05% to about 37% of the weight of the final composition.

If desired, other ingredients, such as diluents, stabilizers and anti-adherents, conventionally used for pharmaceutical formulations may be included in the present formulations. Optional ingredients include coloring and flavoring agents which are well known in the art.

The invention is further described by means of the following examples, which are not intended to limit the scope of the claimed invention in any manner.

### EXAMPLE 1

A bi-layer tablet was formulated for twice daily oral administration, comprising of an immediate release layer containing 5 mg loratadine and 220 mg naproxen sodium, and an extended release layer containing 120 mg pseudoephedrine hydrochloride.

The immediate release layer consisted of loratadine and naproxen sodium combined with various acceptable pharmaceutical excipients using a wet granulation method. Additional excipients were blended extra-granularly prior to compression. More specifically, the formula of the example comprised five intragranular ingredients: naproxen sodium, loratadine, povidone, talc, and croscarmellose sodium which were blended to form the immediate release wet granulation. Extragranular excipients were then blended into the immediate release granulation prior to tablet compression. Extragranular excipients included glyceryl behenate, silicon dioxide, croscarmellose sodium and microcrystalline cellulose.

**Compression Blend for Immediate Release Layer**

| Ingredient | % w/w | mg/finished layer |
|---|---|---|
| Immediate Release Wet Granulation | 72.23 | 252.81 |
| Naproxen Sodium | *87.00* | *220.00* |
| Loratadine | *2.00* | *5.10* |
| *Povidone* | *5*.*50* | *13.90* |
| *Talc* | *3.00* | *7.60* |
| *Croscarmellose Sodium* | *2.50* | *6.30* |
| Microcrystalline Cellulose | 21.27 | 74.44 |
| Croscarmellose Sodium | 2.50 | 8.75 |
| Silicon Dioxide | 2.00 | 7.00 |
| Glyceryl Behenate | 2.00 | 7.00 |
| Total layer weight | 100.00 | 350.00 |

For the extended release layer, pseudoephedrine HCl was combined with various acceptable pharmaceutical excipients using a wet granulation method, and additional excipients were blended extra-granularly prior to compression. More specifically, the example formula comprised of four intragranular ingredients: pseudoephedrine HCl, hydroxypropyl methylcellulose, mannitol and magnesium oxide which were blended to form the extended release granulation. Extragranular magnesium stearate was blended with the extended release granulation prior to tablet compression.

**Compression Blend for Extended Release Layer**

| Ingredient | % w/w | mg/finished layer |
|---|---|---|
| Extended Release Wet Granulation | 99.3 | 470.7 |
| Pseudoephedrine HCl | *25.5* | *120.0* |
| Hydroxypropyl Methylcellulose | *6.8* | *32.0* |
| *Mannitol* | *54.9* | *258.4* |
| *Magnesium Oxide* | *12.8* | *60.3* |
| Magnesium Stearate | 0.7 | 3.3 |
| Total layer weight | 100.0 | 474.0 |

The thus prepared granulations were compressed together to form the bi-layer tablet.

## Claims

1. A pharmaceutical composition comprising naproxen, loratadine and pseudoephedrine wherein the naproxen, loratadine and pseudoephedrine are in a bi-layer tablet and wherein the naproxen and loratadine are in a first, immediate release layer of the bi-layer tablet and the pseudoephedrine is in a second, extended release layer of the bi-layer tablet.

2. The composition as claimed in claim 1, wherein the naproxen is present as naproxen sodium.

3. The composition as claimed in claim 1 or claim 2, wherein the pseudoephedrine is present as pseudoephedrine hydrochloride.

4. The composition as claimed in any one of the preceding claims, wherein the bi-layer tablet contains 5 mg loratadine, 220 mg naproxen sodium and 120 mg pseudoephedrine hydrochloride.

## Patentansprüche

1. Pharmazeutische Zusammensetzung, die Naproxen, Loratadin und Pseudoephedrin umfasst, wobei das Naproxen, Loratadin und Pseudoephedrin in einer Zweischichttablette sind und wobei das Naproxen und Loratadin in einer ersten Schicht mit unverzüglicher Freisetzung der Zweischichttablette sind und das Pseudoephedrin in einer zweiten Schicht mit verlängerter Freisetzung der Zweischichttablette ist.

2. Zusammensetzung gemäß Anspruch 1, wobei das Naproxen als Naproxennatrium vorliegt.

3. Zusammensetzung gemäß Anspruch 1 oder Anspruch 2, wobei das Pseudoephedrin als Pseudoephedrin-Hydrochlorid vorliegt.

4. Zusammensetzung gemäß einem der vorangehenden Ansprüche, wobei die Zweischichttablette 5 mg Loratadin, 220 mg Naproxennatrium und 120 mg Pseudoephedrin-Hydrochlorid enthält.

## Revendications

1. Composition pharmaceutique comprenant du naproxène, de la loratadine et de la pseudoéphédrine, dans laquelle le naproxène, la loratadine et la pseudoéphédrine sont dans un comprimé bi-couche et dans laquelle le naproxène et la loratadine sont dans une première couche, à libération immédiate, du comprimé bi-couche et la pseudoéphédrine est dans une seconde couche, à libération prolongée, du comprimé bi-couche.

2. Composition telle que revendiquée dans la revendication 1, dans laquelle le naproxène est présent sous forme de naproxène sodique.

3. Composition telle que revendiquée dans la revendication 1 ou la revendication 2, dans laquelle la pseudoéphédrine est présente sous forme de chlorhydrate de pseudoéphédrine.

4. Composition telle que revendiquée dans l'une quelconque des revendications précédentes, dans laquelle le comprimé bi-couche contient 5 mg de loratadine, 220 mg de naproxène sodique et 120 mg de chlorhydrate de pseudoéphédrine.
